(19) **Europäisches Patentamt European Patent Office Office européen des brevets**

(11) **EP 2 972 259 B1**

(12) **EUROPEAN PATENT SPECIFICATION**

(45) Date of publication and mention of the grant of the patent:
**08.12.2021 Bulletin 2021/49**

(51) Int Cl.:
**G01N 25/48** *(2006.01)* **G01N 33/15** *(2006.01)*
**G01N 21/05** *(2006.01)* **G01N 21/552** *(2014.01)*
**G01K 17/00** *(2006.01)* **B82Y 15/00** *(2011.01)*

(21) Application number: **14714145.1**

(22) Date of filing: **14.03.2014**

(86) International application number:
**PCT/US2014/027437**

(87) International publication number:
**WO 2014/143658 (18.09.2014 Gazette 2014/38)**

(54) **SYSTEM AND METHOD FOR A MICROFLUIDIC CALORIMETER**

SYSTEM UND VERFAHREN FÜR EIN MIKROFLUIDISCHES KALORIMETER

SYSTÈME ET PROCÉDÉ POUR UN CALORIMÈTRE MICROFLUIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361793545 P**

(43) Date of publication of application:
**20.01.2016 Bulletin 2016/03**

(73) Proprietors:
• **The Charles Stark Draper Laboratory, Inc. Cambridge, MA 02139 (US)**
• **Northeastern University Boston, MA 02115 (US)**

(72) Inventors:
• **FIERING, Jason O. Boston, Massachusetts 02210 (US)**
• **LARSON, Dale Waban, Massachusetts 02468 (US)**
• **TAYLOR, Amy St. Pete Beach, Florida 3306 (US)**
• **SAADI, Wajeeh M. Tampa, Florida 33647 (US)**
• **KOWALSKI, Gregory Beverly, Massachusetts 01915 (US)**
• **SEN, Mehmet Boston, Massachusetts 02215 (US)**

(74) Representative: **Murgitroyd & Company Murgitroyd House 165-169 Scotland Street Glasgow G5 8PL (GB)**

(56) References cited:
**WO-A1-2012/097221**

• **None**

EP 2 972 259 B1

**Description**

BACKGROUND OF THE DISCLOSURE

In general, the invention relates to a microfluidic calorimetry system and a method for using a microfluidic calorimeter. More specifically, the invention relates to a system and method for providing two reagents in microfluidic laminar flow, detecting optical signals transmitted through arrays of nanoholes in a metal film, and processing the optical data to obtain a calorimetry measurement.

Calorimetry is a valuable tool in pharmaceutical research and development, providing information for decision making in drug lead discovery and optimization. Unlike the present high-throughput screening methods used by the industry, such as the affinity sensors, calorimetric analysis can provide very detailed information on the binding interaction between molecules. Calorimetry provides detailed thermodynamic information including the enthalpy and entropy of a reaction. The ability to measure enthalpy and determine entropy allows the drug development team to assess the relative contributions of enthalpy and entropy to a binding reaction. Enthalpy is driven by the number and type of bonds in the binding reaction. Entropy is driven by the geometry of the ligand and the binding site. Understanding the contributions of enthalpy and entropy is critical in drug development because it allows for the selection of compounds that are more readily optimized. Specifically, reactions that are enthalpy-driven tend to be favored due to their enhanced selectivity and reactivity.

With current technology, initial high throughput screening and the first candidate drug selection is performed by affinity analysis. Only after the set of candidate drugs has been narrowed down to select few, candidates are analyzed by the two currently available calorimetry techniques, Differential Scanning Calorimetry and Isothermal Titration Calorimetry, to measure the thermochemical properties of a reaction. The limitations of the current generation of calorimeters include:

- Inadequate sensitivity for reactions with a low change in enthalpy

- Large amount of protein required (0.5mg to 5mg)

- Low experimental throughput because of both long experiment run times (60 to 90 minutes) and the need to sequentially run controls to assess the significance of the confounding effects. The potential confounding effects primarily include heating due to the mixing of dissimilar sample media (buffers with different pH, ionic strength, and solvents), the presence of DMSO from compound storage, and solvation.

Furthermore, compounds with poor solubility frequently generate hits in high throughput screens. Unfortunately, the concentrations of these compounds required to meet the mass requirement for reagents (protein and its ligand) are often above the solubility limit. As a result, calorimetry studies on the interactions of these compounds with their targets cannot be done. Paradoxically, additional synthetic/medicinal chemistry is required before calorimetry can be used, but this chemistry work cannot be justified without the calorimetry data. The outcome of this is that potentially promising compounds are not pursued. The ability to analyze smaller amounts of reagents would reduce this need for concentration.

Beyond pharmaceutical analysis, calorimetry is also valuable in many branches of materials science and chemistry. For example, calorimetry is useful for highly reactive or explosive compounds testing used in the design of chemical processes and safety equipment.

WO2012/097221 discloses a microfluidic calorimeter system including a calorimetry apparatus and a processor in connection with the apparatus. The apparatus includes a microfluidic laminar flow channel connected to two inlets for flowing fluid into the laminar flow channel. Below the laminar flow channel is a plurality of microscale temperature sensors at known positions in the channel. The processor is in connection with the discrete temperature sensors and determines a calorimetry measurement based on local temperatures derived from data output by the microscale temperature sensors and the respective positions of the sensors in the channel.

SUMMARY OF THE DISCLOSURE

There is therefore a need in the pharmaceutical industry for a system and method for microfluidic calorimetry. Extraordinary optical transmission, a physical phenomenon, related to surface plasmon resonance, can be harnessed to produce an apparatus for determining temperature change of a chemical reaction occurring in microfluidic laminar flow. In addition to solving the aforementioned needs for such a calorimeter, the microfluidic calorimeter disclosed herein uses a stationary laminar flow with numerous benefits over traditional calorimetry methods. First, the exact volume of the reagents need not be known. Second, flowing the reagents and observing the reaction along the channel allows the reaction to be observed as it progresses, since the reagents continue to diffuse and react as they flow. Third, since the reaction and diffusion regions are stationary in space, which implies that the heat released at a location along the channel should remain constant for the duration of the test, the collected data can be integrated over time to reduce noise and error in the data.

According to one aspect of the invention, there is provided a method for calorimetry according to claim 6.

In some implementations, the method also includes analyzing portions of the calorimetry measurement, which approximates a second calorimetry measurement made when the reagent was flowing at steady state.

In some implementations, the method includes injecting a second reagent bolus into the first flow channel via the fluid injector, such that the second reagent comes into contact with the second fluid in the microfluidic flow channel. A second calorimetry measurement over a plurality of reagent concentrations is then made as the second reagent bolus flows through the micro fluidic flow channel.

In some implementations, a reaction between the reagent and the second fluid causes a temperature change. In certain implementations, the plurality of microscale sensors are temperature sensors and detect the temperature change. The temperature sensors are nanohole arrays in a metal layer disposed below the laminar flow channel. The nanohole arrays are surrounded by dielectric mirrors. In some implementations, a layer is disposed between the metal layer and the laminar flow channel for transferring heat from a fluid in the laminar flow channel to the surface of the metal layer.

In some implementations, when calculating a calorimetry measurement, at least one enthalpy of the reaction, a binding constant of the reaction, a Gibbs free energy value, a change in free energy, an entropy value, and a change in entropy is calculated.

In some implementations, the volume of the reagent bolus is between about 10 nL and about 250 nL. The volume of second fluid in the microfluidic laminar flow channel is between about 4 and about 10 times greater than the volume of the reagent in the micro fluidic laminar flow channel when the calorimetry measurement is made. In some implementations, the first fluid is selected such that it does not react with the reagent. In certain implementations, the method also includes selecting a flow rate of at least one of the first fluid and the second fluid based on a diffusivity of the fluids.

According to another aspect of the invention, there is provided a system for calorimetry according to claim 1.

In some implementations, the fluid injector includes a proximal end coupled to a proximal end of the first inlet and a distal end coupled to a distal end of the first inlet. The system also includes a valve coupled to the proximal end of the fluid injector that is configured to selectively introduce a fluid into the fluid injector to eject a reagent stored in the fluid injector into the first inlet. The system includes a second fluid inject coupled to the second inlet. The fluid injector is configured to hold between about 10 nL and about 250 nL. In some implementations, the fluid injector is configured to hold between about 0.25 and about 10 times less volume than the micro fluidic laminar flow channel.

The plurality of microscale sensors are temperature sensors configured to detect temperature changes caused by the reaction of the first fluid and the reagent. The temperature sensors are nanohole arrays in a metal layer disposed below the laminar flow channel. The nanohole arrays are surrounded by dielectric mirrors. In some implementations, the system also includes a layer disposed between the metal layer and the laminar flow channel for transferring heat from a fluid in the laminar flow channel to the surface of the metal layer. In certain implementations, the plurality of microscale sensors are optical sensors. The optical sensors are surface plasmon sensors whose response is measured by at least one of a photomultiplier, a charge-coupled device, and a photodiode.

In some implementations, the system includes a microscope configured to take micrographs of the microfluidic laminar flow channel. In some implementations, the system includes an array of microscale sensors disposed at specific distances from the start of microfluidic laminar flow channel.

The processor is configured to calculate a calorimetry measurement responsive to a data set collected by the plurality of microsensors. The calorimetry measurement includes calculating at least one enthalpy of the reaction, a binding constant of the reaction, a Gibbs free energy value, a change in free energy, an entropy value, and a change in entropy.

The processor is also configured to calculate an estimate of the reagent's concentration at a plurality of specific sensor positions and at a plurality of specific time points after the injection of the reagent. The processor is also configured to compare the calorimetry measurement to a calorimetry measurement made under steady state flow conditions, in some implementations.

In some implementations, the system includes a controllable, first and second fluidic pumps configured to flow fluid into the inlets of the device. In some implementations, a rate at which the second fluidic pump flows the second fluid into the second inlet is controllable.

BRIEF DESCRIPTION OF THE DRAWINGS

The skilled artisan will understand that the figures, described herein, are for illustration purposes only. It is to be understood that in some instances various aspects of the described implementations may be shown exaggerated or enlarged to facilitate an understanding of the described implementations. In the drawings, like reference characters generally refer to like features, functionally similar and/or structurally similar elements throughout the various drawings. The drawings are not necessarily to scale, emphasis instead being placed upon illustrating the principles of the teachings. The drawings are not intended to limit the scope of the present teachings in any way. The system and method may be better understood from the following illustrative description with reference to the following drawings in which:

The system and method may be better understood from the following illustrative description with reference to the following drawings in which:

Figure 1 is block diagram of a system for microfluidic calorimetry, according to an illustrative implementation of the disclosure;

Figure 2 is an exploded solid model of an apparatus for use in a system of microfluidic calorimetry, according to an illustrative implementation of the disclosure;

Figure 3 is a diagram of an array of nanoholes used for optical transmission, according to an illustrative implementation of the disclosure;

Figure 4a is a graph of optical transmission versus wavelength for multiple dielectric constants through an exemplary array of nanoholes;

Figure 4b is a graph of optical transmission versus dielectric constant through an exemplary array of nanoholes;

Figure 5 is a diagram of laminar flow channel with inlets and nanohole arrays;

Figure 6a, 6b, and 6c are section views of a laminar flow channel for use in a system of microfluidic calorimetry, according to various illustrative implementations of the disclosure;

Figure 7A is a diagram of a laminar flow channel with an injection valve for use in a microfluidic calorimetry system, according to an illustrative implementation of the disclosure;

Figure 7B is a diagram of the reaction chamber of the microfluidic calorimety system illustrated in Figure 7A, according to an illustrate implementation of the disclosure;

Figures 7C-7E are a series of difference maps of reagent concentration measures generated using a laminar flow channel similar to the flow channel of Figure 7A, according to an illustrative implementation of the disclosure;

Figure 8 is a flowchart for a method of using the microfluidic calorimetry system of Figure 1, according to an illustrative implementation of the disclosure;

Figure 9 is a flowchart for a method of processing optical data received from the microfluidic calorimetry system of Figure 1 to obtain calorimetric measurements, according to an illustrative implementation of the disclosure;

Figure 10 depicts a simulated temperature, according to an illustrative implementation of the disclosure.

Figure 11 depicts graphs illustrating simulation results for heat loss coefficient, temperature, and mass fractions at specific positions across the width of a laminar flow channel, according to an illustrative implementation of the disclosure.

Figure 12 is a graph illustrating the trend between the number of compounds versus time in a pharmaceutical research and development process;

Figure 13 is a flowchart for a method of pharmaceutical research and development utilizing the microfluidic calorimetry system of Figure 1, according to an illustrative implementation of the disclosure.

DETAILED DESCRIPTION

To provide an overall understanding of the disclosure, certain illustrative implementations will now be described, including systems and methods for microfluidic calorimetry.

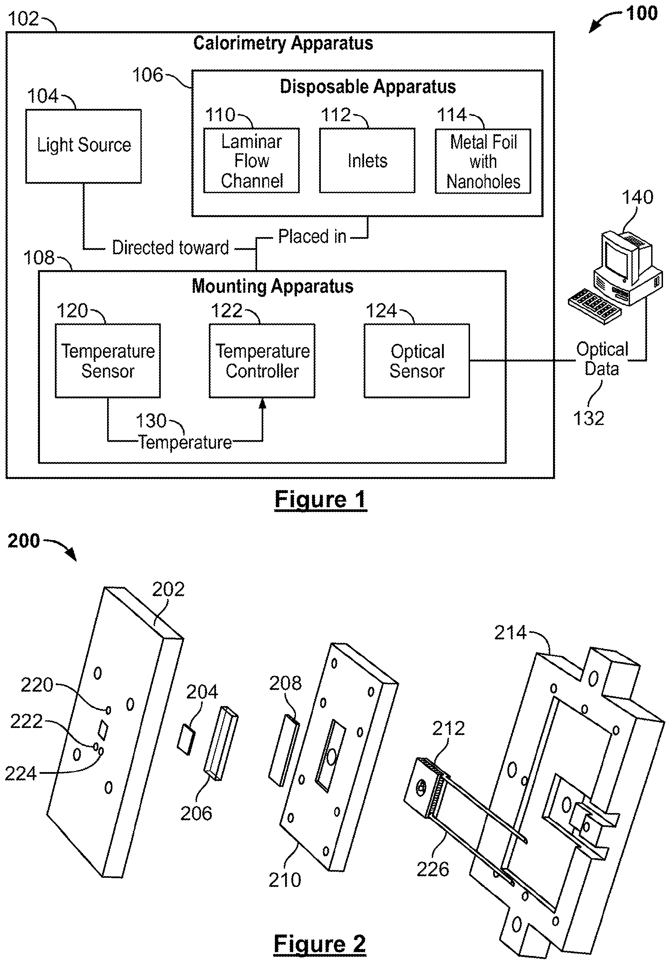

Figure 1 is a block diagram of a system for microfluidic calorimetry 100, according to an illustrative implementation of the disclosure. The system 100 is used to detect temperature change and other calorimetry measurements from a micro-scale chemical reaction. A user runs an experiment in the microfluidic calorimeter by flowing two reagents into and through a laminar flow channel, causing the reagents to react at their diffusion interface. An optical sensor detects Extraordinary Optical Transmission (EOT) signals, which are affected by the temperature change of the flowing solution due to the reaction. The EOT signals are emitted through nanoholes in a metal film below the laminar flow channels. A computer processes the EOT signals to obtain the temperature change and other calorimetric measurements.

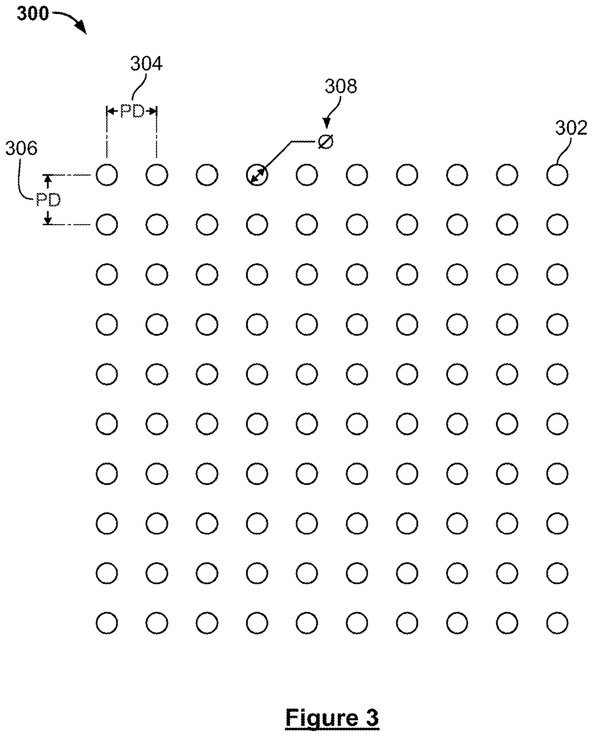

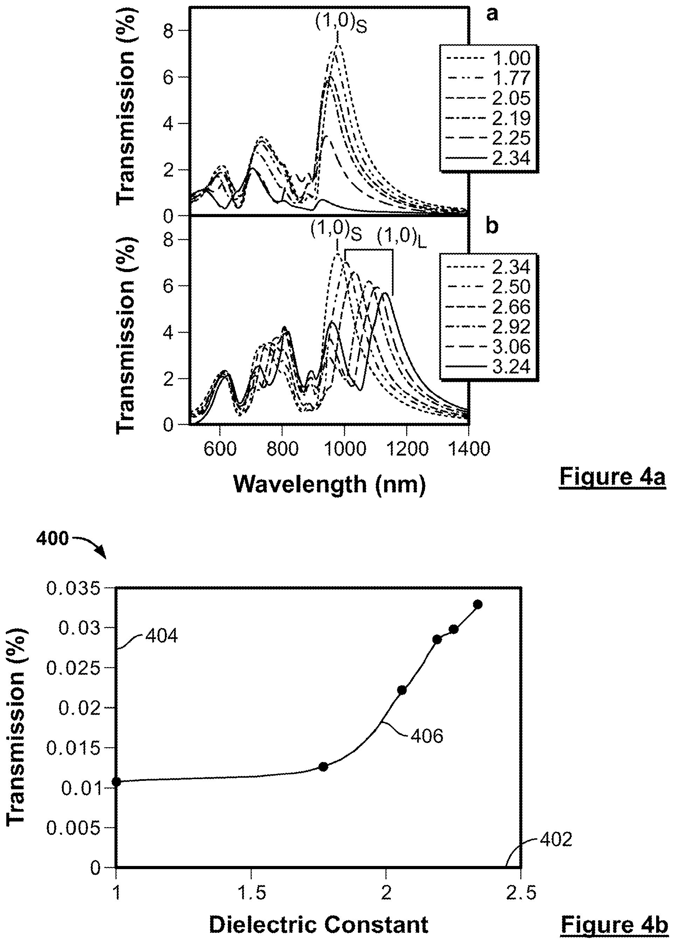

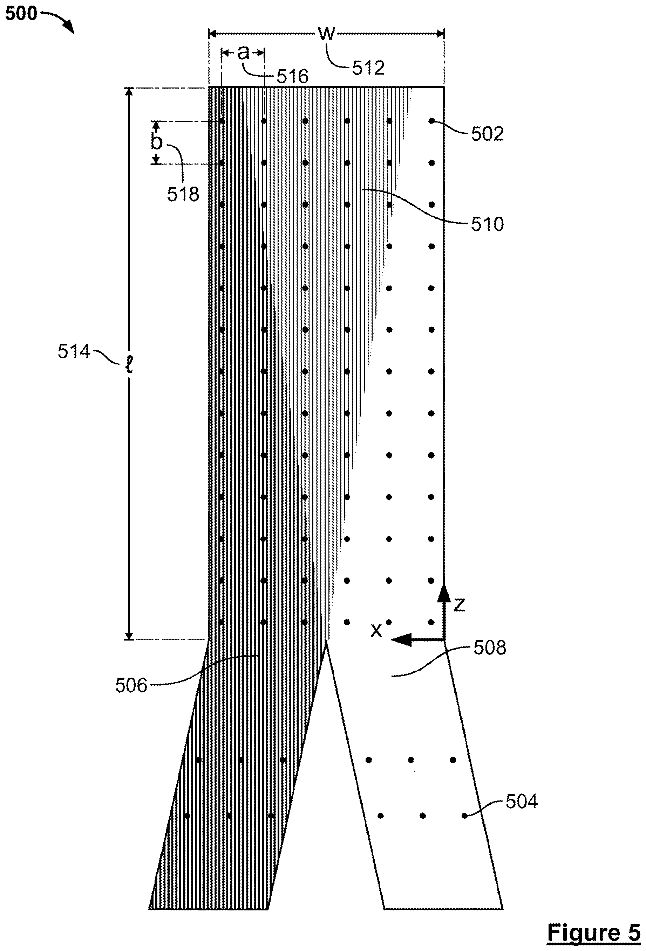

The system consists of a calorimetry apparatus 102 and a processor 140. The calorimetry apparatus 102 consists of a light source 104, a disposable apparatus 106, and a mounting apparatus 108. The disposable apparatus consists of a laminar flow channel 110, inlets 112, and a metal foil with nanoholes 114. The two reagents enter the laminar flow channel 110 from inlets 112. The arrangement of the laminar flow channel and inlets will be discussed in more detail with respect to Figure 5. Below the laminar flow channel is a metal foil 114 with a plurality of arrays of nanoholes used in optical transmission sensing. The heat of the liquid in the laminar flow channel 110 directly above the nanohole arrays affects the surface plasmon resonance (SPR) in the metal foil, which thereby affects light transmission through the nanoholes. The principles of nanohole array sensing are discussed in greater detail with respect to Figures 3 and 4.

The disposable apparatus 106 is placed in a mounting apparatus 108 which consists of a temperature sensor 120, a temperature controller 122, and an optical sensor 124. The temperature sensor 120 and temperature controller 122 are used to maintain a fairly constant system temperature to prevent environmental temperature fluctuations from impacting the experiment. The temperature sensor 120 detects the environmental system temperature 130 and sends it to the temperature controller 122. Based on the current environmental system temperature 130, the temperature controller 122 either heats or cools the system, or takes no action if the temperature is near enough to the set temperature. In one implementation, the environmental temperature is permitted to vary by 0.0002 °K, but depending on the amount of heat released by the reaction, the required accuracy, and the precision of the temperature sensor, the environmental temperature may be held within smaller or larger ranges. The temperature 130 may also be sent to the system processor 140 for use in calibration of the calorimetry apparatus or for use as a reference temperature in calculating calorimetric values.

The optical sensor 124 detects light emitted through the nanoholes in the metal foil 114. The optical sensor 124 may be a photomultiplier, a photodiode, a charge-coupled device (CCD) or other image sensor, or any other apparatus capable of detecting light with sufficient sensitivity to distinguish nanohole arrays from one another. The optical sensor sends the optical data received through the nanoholes to processor 140.

The calorimetry apparatus 102 also includes a light source 104 which emits light directed toward the metal film with nanoholes 114. In one implementation, the light source 104 emits light which travels through the laminar flow channel 110 and onto the metal foil at incident angle 8. The light source may be a single wavelength laser. Alternatively, the light source may be a broadband source, and a monochromator or optical filters may be used to deliver a monochromatic beam of light, or spectrometry techniques may be used in detection.

Figure 2 is an exploded solid model 200 showing parts of the mounting apparatus 108 and the disposable apparatus 106 in an exemplary implementation of a system for microfluidic calorimetry. From left to right, the solid model 200 depicts a plastic cover 202, coverglass 204, flow cell 206, nanohole array chip 208, mounting tray 210, heating unit 212, and plastic base 214. The plastic cover 202, mounting tray 210, and plastic base 214 support and protect the apparatus. They are the main structural components of the mounting apparatus 108. Flow cell 206, part of the disposable apparatus 106, contains features that comprise walls of the laminar flow channel 110 and the inlets 112. The flow cell 206 is molded from a polydimethylsiloxane (PDMS) mixture that insulates the flow cell from the top, but another similarly insulative material that can be molded with sufficient precision may be used. A 3D solid object printer may fabricate a mold used to create the PDMS flow cell. Alternatively, photolithography may be used to form the flow cell 106. The access holes 220, 222, and 224 allow tubing access to the flow cell. Access hole 220 provides access to the laminar flow channel 110 so that fluids may be removed after flowing through the flow channel, and access holes 222 and 224 provide access to the two inlets to introduce reagents into the inlets. The tubing that enters the inlets 112 through the access holes 222 and 224 connect to a means of controlling injection volume and speed into the inlets 112, such as syringes with syringe pumps. The mounting elements 202, 210, and 214 may be constructed using a 3D solid object printer or other suitable manufacturing method. After assembling the calorimetry apparatus, the plastic cover 202 and plastic base 214 are tightened using screws or other clamp assembly, not shown, to hold the elements in contact with each other.

The nanohole array chip 208 consists of a conductive surface, in this case the metal foil with nanoholes 114, atop a transparent surface, such as a glass substrate, which also serves as an insulator. The types and properties of suitable metals for use in nanohole sensing and further details about designing and producing nanohole array chips are discussed in U.S. Patent No. 7,318,907, the contents of which are incorporated herein in its entirety. In an exemplary implementation, a thin (e.g. 25 nm) layer of chromium or other metallic bonding agent is first evaporated onto the glass substrate. A thicker (e.g. 105 nm) layer of gold is then evaporated atop the chromium. In the alternative, the metal foil could be made of gold, silver, aluminum, beryllium, rhenium, osmium, potassium, rubidium, cesium, rhenium oxide, tungsten oxide, copper, titanium, or another suitable metal. Once the metals have been deposited, nanohole arrays are milled into the metal using, for example, photolithographic techniques, electron beam lithographic techniques, a focused ion beam (FIB), or other methods. For a FIB with a current of IpA, 15 seconds is necessary to mill through the metal layers. As depicted in Figure 1, the nanohole array chip 208 may be part of the disposable apparatus. The nanohole array chip 208 may be attached to the flow channel, or it may be a separate disposable part that mounting tray 210 aligns with the mounting apparatus 108. In this implementation, the fluids in the laminar flow channel 110 directly contact the metal foil when the apparatus is assembled and clamped.

Heating unit 212 consists of a thermoelectric heater and at least one temperature sensor, such as a thermistor. Wires 226 connects the heating unit 212 to a heat controller (not shown), which controls the thermoelectric heater based on the temperature from the thermistors. The heating unit 212 may also be able to cool the apparatus. Optical sensor 124, not shown on Figure 2, is below the plastic base 214. The heating unit 212 and mounting elements 210 and 214 have holes in their center so that light passing through the nanoholes can reach and be measured by the optical sensor 124. The hole in the heating unit does not substantially affect the temperature consistency throughout the laminar flow channel 110.

The disposable apparatus 106 may contain at least a second laminar flow channel (not shown) that may be fluidly connected to the same set of inlets or to one or more additional inlets for a multiplexed design that permits more than one experiment on a single chip. Another set of nanohole arrays on the metal foil 114 or a second metal foil is below the second laminar flow channel. The mounting apparatus may be configured to run multiple experiments at the same time or perform experiments one at a time. If experiments are to be performed one at a time, the mounting apparatus may have a single optical sensor that moves from flow cell to flow cell as tests are run at each flow cell. An experiment performed in one laminar flow channel in the multiplexed apparatus could differ from an experiment performed in a different laminar flow channel in at least one of environmental temperature, a reagent material, a solvent material, a reagent concentration, and reagent flow rate.

Figure 3 is a diagram of a single ten-by-ten nanohole array 300. The interaction of incident light from the light source and surface plasmon resonance (SPR) excited by the light source causes extremely efficient light transmission through the nanoholes, called extraordinary optical transmission (EOT). Previous studies and experimental results have shown that the behavior of EOT signal due to changes in concentration or temperature is dependent on a variety of nanohole parameters, such as diameter of the nanoholes, shape of the nanoholes arrangement (e.g. rectangular vs. circular arrangements), size of the nanohole array, positioning of the nanohole arrays, etc. In this implementation, the nanohole array 300 is formed of 100 nanoholes 302 positioned in a square, but the number and arrangement of nanoholes may be different; for example, three-by-three and nine-by-nine nanohole arrays are also commonly used. The nanoholes may be arranged in a different pattern, such as a hexagonal, linear, or bull's eye pattern. The pitch distances 304 and 306 between the nanoholes, measured from the center of one nanohole to the center of a neighboring nanohole, are labeled PD. The horizontal pitch distance 304 and vertical pitch distance 306 are the same. In one implementation, the pitch distance PD is 350 nm, and diameter of each nanohole 308 is 150 nm (Ji et al., 2008). The choice of 150 nm is decided by the easy fabrication of relatively large holes using Focused Ton Beam (FIB) and electron-beam lithography (Ji et al., 2008). The hole diameter may range from 130 nm to 290 nm, or smaller holes may be used. The pitch distance is dependent on the wavelength of the incident light, the geometric pattern of nanoholes, and the dielectric constants of the metallic film and the solvent above the metallic film. Pitch distance may range from 220 nm to 540 nm. The pitch is a function of the wavelength of the light being used and the geometric pattern being used.

Each nanohole array 300 may be surrounded by dielectric mirrors, such as Bragg mirror groves, to reflect and confine the surface plasmon energy associated with a transmission peak of the nanohole array. This creates both constructive and destructive interference effects, thereby enhancing or decreasing the transmission at certain wavelengths. Use of Bragg mirrors for nanohole sensing applications is discussed in greater detail in Lindquist et al. (2009), the contents of which are incorporated herein in its entirety.

The percentage of transmitted light in EOT transmission is affected by the wavelength of the incident light, as shown in Figure 4a, which shows the transmission percentage versus wavelength for multiple fluid dielectric constants (Krishnan, A. et al. 2001). For a particular dielectric fluid layer with dielectric constant £1 and a metal foil with dielectric constant $\varepsilon_2$, light having angle of incidence $\theta$, metal foil having lattice constant $a_0$, and a constant $\gamma$, the resonance wavelength peak is as follows:

$$\lambda_{peak} = \frac{a_0}{\gamma}\left[\left(\frac{\varepsilon_1\varepsilon_2}{\varepsilon_1 + \varepsilon_2}\right)^{1/2} - sin(\theta)\right]$$

**Equation 1**

Equation 1 indicates that a change in resonance wavelength at a peak is based on the change in the dielectric constant of the dielectric fluid layer, as all other variables are constant during a chemical reaction observed in the calorimetry system. The change in dielectric constant depends on changes in temperature, pressure, and concentration of a material. Relating the dielectric constant to changes in temperature and pressure, while holding concentration constant and defining:

$$\Phi = \frac{\rho}{\rho_0} = exp(-[\beta(T - T_0) - k_T(P - P_0)])$$

wherein p is density, T is temperature, $\beta$ the coefficient of volumetric expansion, T is temperature, $k_T$ is the isothermal compressibility factor, and P is pressure, gives:

$$\lambda_{peak} = \frac{a_0}{\gamma} \left( \frac{\epsilon_2 \left[ \frac{1+2C_0\rho_0\Phi}{1-C_0\rho_0\Phi} \right]}{\frac{1+2C_0\rho_0\Phi}{1-C_0\rho_0\Phi} + \epsilon_2} - sin(\theta) \right)$$

**Equation 2**

wherein constant concentration C is:

$$\left[ \frac{n_0^2 - 1}{n_0^2 + 2} \right] \frac{1}{\rho_0}$$

The references values of temperature and pressure conditions are represented by $n_0$ and Po. As shown in Figure 4a, experimental results show that as the dielectric constant $\epsilon_2$ the fluid changes, both the peak wavelength and the percentage of transmission at the peak wavelength changes. Moreover, for a single wavelength, transmission percentage, which is measured by the optical sensor 124, changes as the dielectric constant of the fluid changes.

This is more clearly shown in Figure 4b, a graph showing percentage of incident light transmitted through a subwavelength nanohole array versus the dielectric constant of the material adjacent to the nanoholes (Krishnan, A. et al. 2001). Since the dielectric constant depends on the temperature, pressure, and concentration of the material, an EOT sensor can therefore be used as a temperature sensor.

Figure 5 is a diagram of laminar flow channel 110 and inlets 112 with nanohole arrays 502 and 504. Flow is fully developed in the channel and is at steady state condition, which is not according to the invention. The left inlet contains a first reagent 506, shown in a dark stripe. The right inlet contains a second reagent 508, shown in solid white. For an exemplary pharmaceutical test, the fluid flowed through one inlet contains a drug and the fluid flowed through the other inlet includes a protein to which the drug mayor may not bind. Other kinds of reagents may be used for other types of calorimetry studies. The lighter striped region 510 shows the diffusion region at the interface of the two reagents in which the reaction is occurring. The shape of this diffusion region will depend on the flow rates of the reagents and the diffusivity of the fluids; more diffusive reagents or will have a wider diffusion region than less diffusive reagents. The reagents may be given two different flow rates to control the shape or position of the region. An acceptable range for the flow rate in the channel is from 0.0005 m/s to 0.05 m/s, or from 0.15 µL/min to 15 µL/min. Although for illustration the inlets in Figure 5 are shown entering the laminar flow channel at an angle, it is preferable that before entry into the laminar flow channel they are substantially parallel to each other and in line with the laminar flow channel so that the fluids have no x-velocity upon entering laminar flow.

The diffusion region 510 is stationary over time, providing several key advantages over traditional calorimetry methods. As reagent volume is added to the channel, reagents continue to flow through the channel so at a given nanohole under the diffusion region 510 the fluid above it has a substantially similar concentration and heat of reaction for the duration of the test. This allows an observer to integrate the data over time, reducing noise and error in the measurements. Additionally, since the diffusion region is spread out along the length of the channel, multiple stages of the reaction are observed simultaneously, allowing detailed observations for how the reaction progresses with time. Furthermore, since the ratio of the left reagent to the right reagent varies across the width of the diffusion region, nanoholes across the width of the channel observe the reaction with varying relative concentrations of reagents.

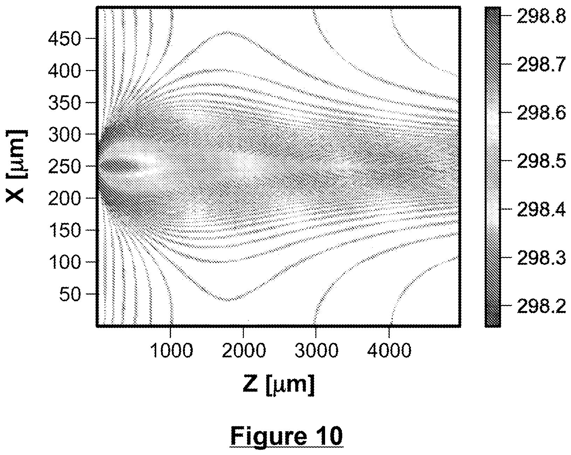

The width of the laminar flow channel 512, labeled w, and length of the channel 514, labeled 1, are not to scale, as the length 1 is typically much longer than the width w. In one implementation, w is 500 $\mu$m and length is 5000 $\mu$m to provide a sufficiently large surface area to observe substantial interdiffusion of reagents and generation of reaction products. More generally, the width could be typically in the range of 50 to 1000 $\mu$m, and the length could be in the range of about 500 to 10000 $\mu$m. The channel width and length are bounded from above by the desired reagent volume consumed and by the need to maintain laminar flow, and from below by the minimum separation of nanohole arrays and desired resolution of nanohole arrays, i.e. the number of nanohole array sensors. The height of the laminar flow channel, in one implementation, is 10 $\mu$m, but may be typically in the range of 1 to 30 $\mu$m. A smaller height will reduce reagent volume consumed and may improve accuracy and sensitivity, since variations in temperature across the height of the channel will be minimized. However, practical considerations such as fabrication tolerances and inlet pressure may set a lower bound on channel height. Reagent volumes consumed are determined by the internal geometric volume of the laminar flow channel. The reagent volume of each of the two components will be at least equal to one half the product of the length, width, and height of this channel. In practice, typically 2 to 10 times this product will be needed to stabilize flow and perform a measurement. Other considerations in designing the laminar flow channel are the flow rates needed for laminar flow and the diffusivity of the reagents. The channel width and length will vary depending at least on these characteristics. Nanohole arrays 502 are equally spaced horizontally by distance 512, labeled a, and vertically by a distance 518, labeled b. As shown, a and b are equal, but they need not be. Furthermore, the nanohole arrays 502 need not be equally spaced throughout the laminar flow channel 110. For example, in the horizontal direction, nanohole arrays 502 may be more concentrated near the center of the channel where the reagents are reacting. In the vertical direction, nanohole arrays 502 may be concentrated near the inlets 112 where the reagents begin to react. While placing nanohole arrays 502 near the reaction is important to capture the temperature change, it may also be useful to place nanohole arrays 502 near the edges of the laminar flow channel 110 to take baseline measurements of the fluids whose temperature has not been affected by the reaction. Similarly, it may be useful to place nanohole arrays 504 or other types of temperature or concentration sensors in the inlets 112 to take baseline measurements of the reagents before any reaction or diffusion. One method for determining spacing of the nanoholes 502 in the laminar flow channel is discussed in more detail with respect to Figure 10.

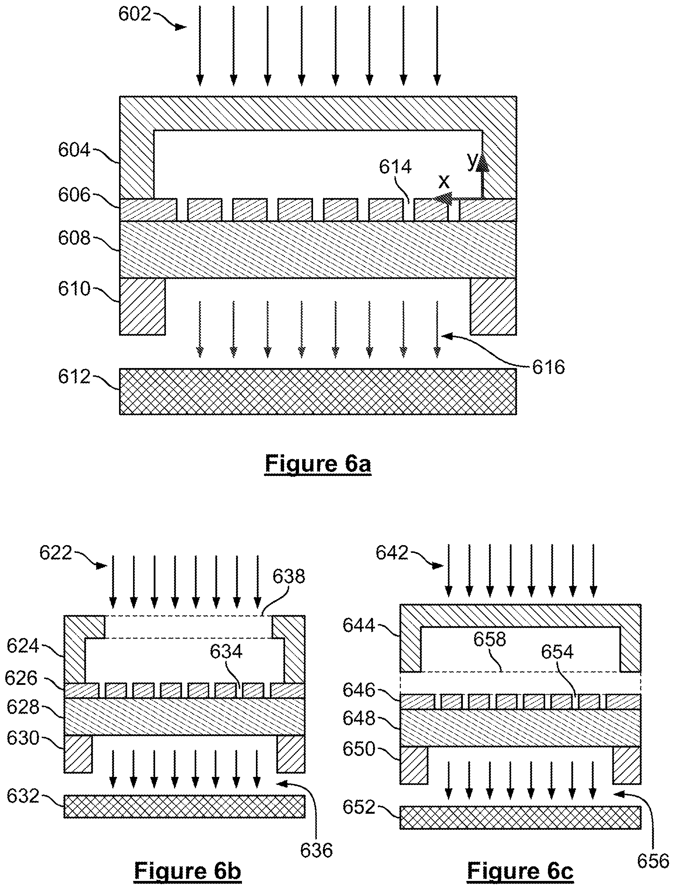

Figure 6 shows various section views of laminar flow channels for microfluidic calorimetry. Figure 6a shows laminar flow channel 600 consisting of PDMS channel 604, metal foil 606 with nanoholes 614, glass substrate 608, heating unit 610, and optical sensor 612. Reagents travel through the rectangle bounded by the PDMS channel 604 and the metal foil 606. Incident light 602 travels into the channel through the PDMS layer 604, hitting the metal foil 606. This excites surface plasmons at the top of the metal foil, which interact with the light creating EOT 616 that travels through the glass substrate 608 and to the optical sensor 612. As mentioned previously, the optical sensor may be a photomultiplier, a photodiode, a charge-coupled device (CCD) or other image sensor, or any other apparatus capable of detecting light with sufficient resolution to distinguish nanohole arrays from one another. Heating unit 610, similar to heating unit 212, again does not interfere with the EOT 616. The elements of the laminar flow channel and other equipment are not to scale.

Figure 6b is similar to Figure 6a, but it includes optical window 638 at the top of the laminar flow channel. This allows the reaction to be observed from above. It also allows the sides of the laminar flow channel to be constructed by a material, such as a silicate, that would otherwise interfere with the light source, as the light source would travel through the optical window rather than through the interfering material. Access to alternate materials for manufacturing the flow channel allows greater design precision and therefore a smaller laminar flow channel volume with more material options.

Figure 6c is also similar to Figure 6a, but in this case laminar flow channel 660 also contains a dielectric heat transfer layer 658 disposed above the metal foil 646 and below the PDMS channel 644. As the nanohole arrays can only sense temperature changes a very small distance above their surface, e.g. 100 nm, the heat of the reaction must travel through the heat transfer layer 658, or the heat transfer layer must be extremely thin. If the glass substrate coated in the metal foil is to be reused for more than one calorimetry test, the heat transfer layer 658 would be part of the disposable apparatus 106 from Figure 1 to protect the reusable metal foil from the reagents and the reaction. Layer 658 may be composed of, for example, vapor deposited silicon dioxide, silicon nitride or spin coated or vapor deposited polymers such as PDMS, acrylic, or polycarbonate.

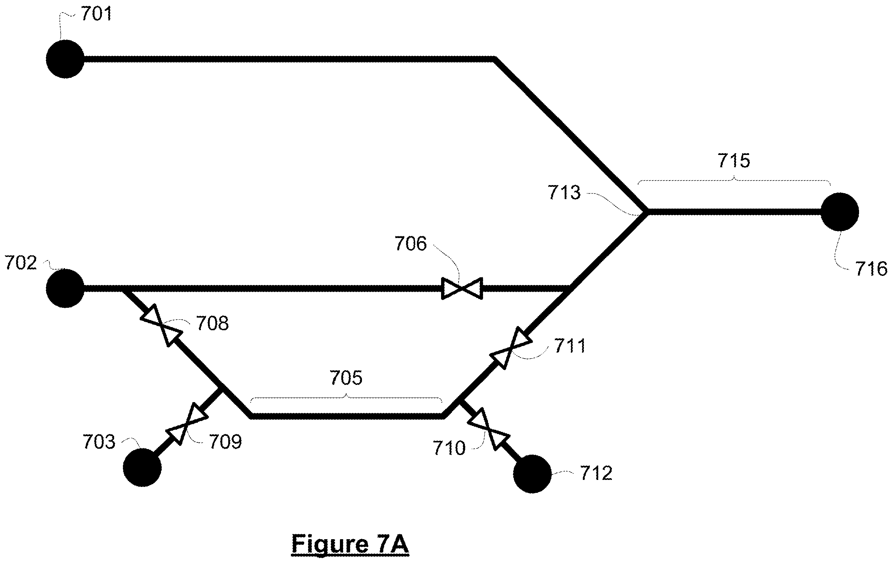

Figure 7A shows a laminar flow channel. The laminar flow channel 700 includes a downstream portion, referred to as a reaction chamber 715, a first fluid inlet port 701, a buffer inlet port 702, and an outlet port 716. The laminar flow channel 700 also includes a reagent inlet port 703, through which a bolus of the reagent can be injected into a reagent reservoir 705. The laminar flow channel 700 also includes a number of valves 706, 708, 709, 710, and 711. To fill the reagent reservoir 705, the device is primed with a fluid such as the buffer fluid. Once primed, the valves 706, 708, and 711 are closed. Valves 709 and 710 are opened and a bolus of the reagent is pumped into the reagent reservoir 705 from the reagent inlet port 703. As the reagent bolus enters the reagent reservoir 705, the volume of the bolus displaces a portion of the priming fluid in the reagent reservoir 705. The excess priming fluid flows out through the outlet port 712. As in Figure 5, the shape of this diffusion region will depend on the flow rates of the reagents and the diffusivity of the fluids. In some implementations, the buffer and/or priming solution can be any suitable aqueous buffer, preferably an optically clear buffer with dielectric properties that are sensitive to temperature.

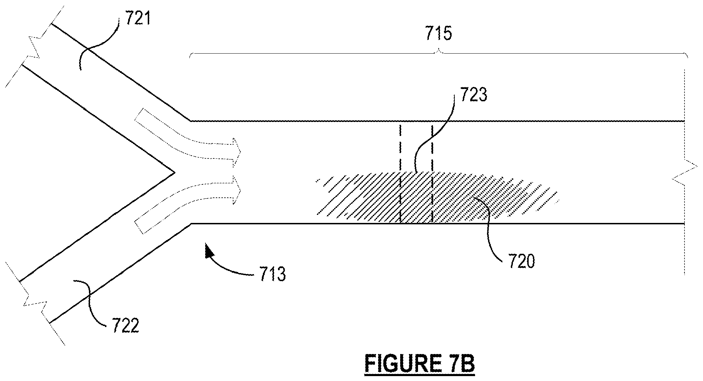

During an experiment, the first fluid and buffer fluid enter the device from inlet ports 701 and 702, respectively. Baseline measurements may be taken and/or calibrations performed while the buffer fluid, which does not react with the first fluid, is flowing. After a laminar flow is initialized, the valve 706 is closed and valve 708 and 711 are opened. As the buffer fluid flows through the reagent reservoir 705 (also referred to as a fluid injector) the reagent bolus is injected into the reaction chamber 715. The injection of the bolus into the reaction chamber 715 is described further in relation to Figure 7B. The device minimizes the amount of reagent used in an experiment by only injecting a bolus of the reagent rather than flowing the reagent through the reaction chamber 715 at steady state flow levels. This is especially beneficial if the reagent is scarce or expensive. In some implementations, the volume of reagent bolus is between about 5 nL and about 300 nL, about 10 nL and about 250 nL, about 10 nL and about 200 nL, about 10 nL and about 150 nL, about 10 nL and about 100 nL, or about 10 nL and about 50 nL. In some implementations, the fluid reservoir 705 is configured to hold between about 0.25 and about 10, about 1.25 and about 7, or about 2 and about 5 times less volume than reaction chamber 715. In one implementation, the reagent is a protein solution, and the right inlet contains a drug, but other reagents may be used for other applications, such as enzymological studies, combustion, control systems, temperature compensation circuits, and the study of explosives. In another implementation, buffer solution is injected from both the first fluid inlet port 701 and the buffer inlet port 702. In some implementations, the device can include more than one reagent reservoirs 705 for the injection of reagents into the fluid. In some implementations, a reagent reservoir 705, and the accompanying inlet and outlet ports and valves, are coupled to the flow channel connected to the first fluid port 701. In some implementations, the flow channels of the device are between about 150 $\mu$m and about 300 $\mu$m wide and between about 15 $\mu$m and about 50 $\mu$m tall. In some implementations, the length of the reaction chamber 715 is between about 3 mm and about 10 mm.

In some implementations, injecting the bolus and/or opening and closing the valves does not disrupt the fluid pressure into the laminar flow channel, which may disrupt the laminar flow. Even after the reagent has been released, flow continues through the injection valve pathway so that the pressure of the fluid behind the reagent is not disrupted.

In this implementation, the reagent may mix with the buffer solution as it leaves the valve. The valve 706 may remain slightly open when injecting the bolus to allow the buffer from the buffer inlet 702 to mix with the reagent as it exits the reagent reservoir 705. In this case, the nanoholes can be used to measure the EOT signals of the reagent solution and determine its concentration.

Pumps may be used in an injection system to regulate flow; for example electrokinetic injection systems and peristaltic injection systems may be used, or any other microscale injection system, such as the injection flow system disclosed in Leach, et al. (2003), the contents of which are incorporated herein in its entirety. In one multiplexed implementation, multiple injection valves are placed on a single inlet so that multiple tests may be run through the same laminar flow channel.

In some implementations, a bolus of reagent can be injected and studied at specific distances in laminar flow channel 700 from the Y connection 713. For example, a bolus of reagent 708 can be injected and then measured at distances about 0.10 mm to about 0.15 mm, about 1.50 mm to about 1.60 mm, or about 3.0 mm to about 3.2 mm away from the Y connection 713.

In some implementations, the small scale of the bolus and the bolus' non-uniformities significantly influence test results. In some implementations, the effects of dispersion, bolus profile, and other non-uniformities that can affect experimental outcome are taken into account by comparing the concentration profile in the bolus to the concentration profile of the same reagent under steady-state continuous flow. In some implementations, a user can create a single concentration profile of the reagent under steady state flow, and then use the concentration profile in subsequent bolus experiments. In certain implementations, this can save on reagent in the subsequent experiments.

In certain implementations, a bolus concentration profile is compared to a steady state concentration profile to determine regions of the bolus concentration profile that approximate the concentration profile of the steady state. In other implementations, the bolus concentration profile is not directly compared to steady state concentration profiles. Rather specifics about the bolus concentration profile are estimated responsive to known flow dynamics of the laminar flow channel 700.

Figure 7B illustrates the Y connection 713 of Figure 7A in greater detail. As described above the first fluid enters the reaction chamber 715 from the channel 721, which is coupled to the inlet 701. The bolus 720 is carried by the buffer fluid and enters the reaction chamber 715 from the channel 722. The reaction between the bolus 720 and the first fluid is measured at the laminar flow interface 723 between the streams of the channels 721 and 722. In some implementations, at the laminar flow interface 723 the reaction between the bolus 720 and the first fluid is comparable to a steady state concentration profile between the bolus and the first fluid at steady state.

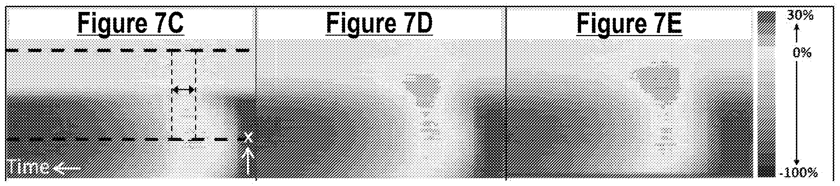

Figures 7C-7E are a series of difference maps comparing the bolus concentration profile to a steady state concentration profile. Generated from an experiment in which a 50 nL bolus of fluorescein was injected into a laminar flow channel with a injector valve similar to the laminar flow channel 700 described above. Figure 7C is a difference maps that compares the concentration profile of the bolus to the concentration profile at steady state when the bolus had traveled 0.11 mm down the laminar flow channel. Figure 7D is a difference maps that compares the concentration profile of the bolus to the concentration profile at steady state when the bolus had traveled 1.54 mm down the laminar flow channel, and Figure 7E is a difference maps that compares the concentration profile of the bolus to the concentration profile at steady state when the bolus had traveled 3.09 mm down the laminar flow channel. In this experiment, the difference maps illustrate the percent difference of the bolus concentration profile and the steady state concentration profile. Figure 7C also includes a dashed box that marks the region of the difference map where the concentration profile of the bolus approximates the steady state concentration profile. Additionally, as each concentration profile illustrates the evolution of the bolus, the length of the region of the difference map where the bolus approximates the steady state provides an approximation of the time measurement of the reaction.

In some implementations, the concentration distribution of the bolus can be predicted in time and space from theoretical calculations and/or simulations, and this information can be used to infer the concentration of reagent at any time point after injection into the inlet 716 and flow channel 700. The predicted concentration may then be used along with temperature measurements to calculate a calorimetric quantity

In some implementations, the concentration profile of the bolus changes as the bolus flows down the laminar flow channel 700. The bolus can interdiffuse with the other fluids in the laminar flow channel 700 and, if the laminar flow channel 700 is long enough, create a homogeneous fluid mixture. As the bolus interdiffuses with its surrounding fluids the relative concentration of the reagent in the bolus diminishes. Using this principle, in some implementations, a single bolus is injected into laminar flow channel 700 and used to study the chemical reaction at a plurality of reagent concentrations with a test fluid. For examples, a bolus of the reagent 708 can be injected into the laminar flow channel 700 at a relatively high concentration level. As the bolus flows through the laminar flow channel 700 it chemically reacts with and/or interdiffuses with the surround fluids resulting in a bolus concentration that is continuously decreasing relative to the initial concentration. In some implementations, a single bolus style experiment, as described herein, can replace a plurality of experiments conducted at steady stated flow and each at a different reagent concentration, therein reducing the amount of reagent required to generate the same data.

In some implementations, the longitudinal spread, or dispersion, of the bolus as the bolus progresses along the length of the reaction chamber 715 is mathematically modeled. In some implementations, the Taylor-Aris theory of hydrodynamic dispersion is used to predict the mass distribution of the bolus as it flows along the length of the reaction chamber 715. The calculation of the dispersion of the bolus enables, given a channel height and width, the calculation of the average concentration at a downstream position in the channel as a function of time. In some implementations, it is assumed that the reaction chamber 715 is shallow enough to allow the concentration of the bolus to be uniform across the height of the reaction chamber 715 at a given position. In this approach, a Peclet number $Pe(x) = v(x)h/D$ is defined such that it varies across the width of the channel according to the velocity $v(x)$ of the fluid flow. The Peclet number is used in calculating the diffusion coefficient:

$$D_{eff}(x) = D\left(1 + \frac{1}{210}Pe(x)^2\right)$$

$$.$$

**Equation 3**

The average concentration of the bolus may be calculated by:

$$c(z,t) = \frac{1}{2w}\int_{-\frac{w}{2}}^{\frac{w}{2}}\left(1 + erf\left(\frac{z - z_0 - v(x)t}{2\sqrt{D_{eff}(x)t}}\right)\right)dx$$

$$.$$

**Equation 4**

In the above equation, the height of the channel is assumed to be uniform and the initial concentration of the bolus is normalized to unity. The position $z_0$ is the location of the concentration step at $t=0$ and erf is the error function.

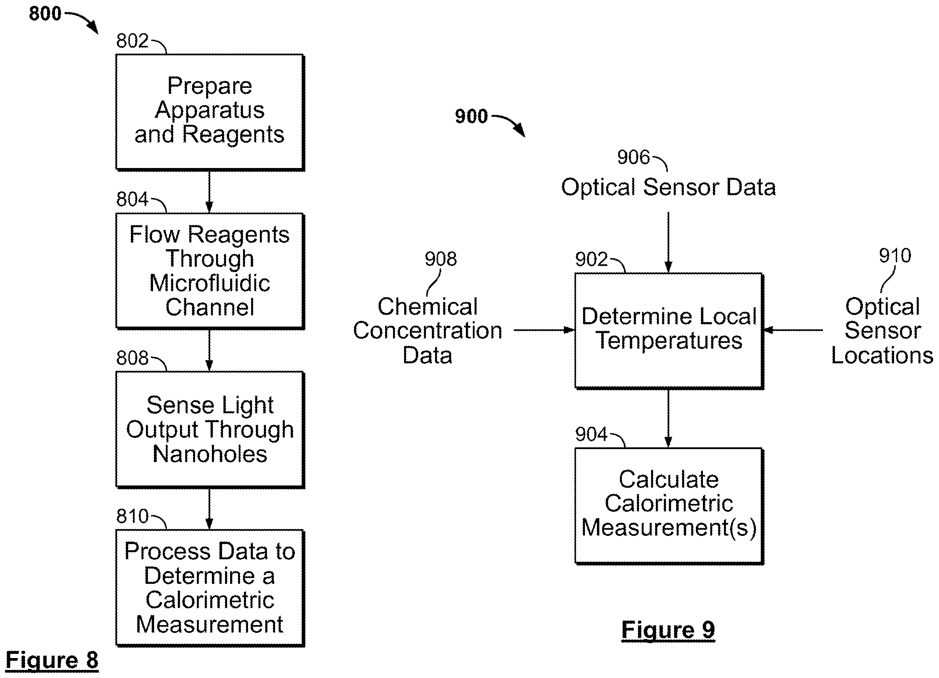

Figure 8 is a flowchart for a method of using a microfluidic calorimeter, especially the calorimeter of Figure 1. First, the apparatus and reagents are prepared (Step 802). This may include putting reagents into injection valves, as discussed with respect to Figure 7, and further preparing buffer fluids. Reagents may be dissolved into or mixed with a solvent. In this case, the heat of the chemical reaction will cause a detectable temperature change in the fluid. Preparing the apparatus includes assembling calorimetry apparatus, e.g. by inserting the disposable apparatus including at least the inlets and laminar flow cell into the mounting equipment so that the optical sensor, nanoholes, and laminar flow channel are properly aligned, with the heating unit in place and not disrupting the path of the light. As applicable, preparation also includes aligning the light source so that it is directed at the nanoholes and, if entering the laminar flow channel from above, the angle of incidence is known. Any tubing to external fluid sources or injection valves is connected. Finally, wiring to an external heat controller and a computer for receiving and processing the data is connected as needed.

Next, the reagents are flowed through the microfluidic channel (step 804). The flow rates are selected prior to beginning the test, and they are controlled by the injection system, which includes the injection mechanism for the buffer solution and any injection valves. Alternatively, the flow rates may be adjusted and/or other calibrations performed as the buffer solution is passing through the laminar flow channel. If both inlets are connected to injection valves that inject the sample after initialization with a buffer solution, release of the reagents from the injection valves must be carefully controlled so that both reagents occupy the laminar flow channel simultaneously.

Once fluids are flowed into the laminar flow channel, the optical sensor senses the light output through the nanoholes and sends the data to a connected computer processor (step 806). The computer processor then processes at least the light emission data to determine a calorimetric measurement of the reaction (step 808). The data processing to determine calorimetric measurements is discussed with respect to Figure 9.

Figure 9 is a flowchart for a method of processing optical data to obtain calorimetric measurements. The computer receives optical data 906 from the optical sensor. Also, the computer has in memory or receives the locations of the optical sensors 910 along the channel. The optical sensor data depends on both concentration and temperature in the relationship $D = TC + Error$, wherein D is a matrix of the optical data over space, T is the temperature matrix, which is convoluted with concentration matrix C, and the error term is experimental measurement noise. Multivariate curve resolution alternating least squares (MCR-ALS) is a deconvolution technique that may be used to iteratively calculate concentration and temperature to best fit the experimental data within given constraints, such as non-negativity, closure, or other hard modeling constraints. Other suitable iterative or non-iterative deconvolution methods may be used. Since the optical data depends on both the concentration and temperature of the fluid, information about the chemical concentration 908 is preferably known in order to determine fluid temperature at the nanosensor array locations from the optical data (step 902). Since the reagents are in laminar flow undergoing diffusion, known mass transport properties of the reagents may be used to determine an initial guess for concentration used as in the deconvolution algorithm to determine temperature. Additionally, nanohole arrays in the laminar flow channel or in the inlets may be used as chemical concentration sensors in locations where the temperature is known. Alternatively, other types of chemical concentration sensors may be placed along the laminar flow channel or inlets to directly measure the concentration. Since the diffusion region is stationary in space, the temperature map and optical data should be stationary over time. Therefore, in order to reduce the noise term, the data may be integrated over time.

Once the local temperatures have been determined, other calorimetric measurements may be calculated (step 904). The x and z directions are shown in Figure 5 at the initial intersection of fluids 504 and 506. The origin (x=0; y=0; z=0) is where the right edge of the

$$\rho c V_z \frac{\partial T}{\partial z} = k \left( \frac{\partial^2 T}{\partial x^2} + \frac{\partial^2 T}{\partial y^2} + \frac{\partial^2 T}{\partial z^2} \right) + \dot{q}(x, y, z)$$

**Equation 5**

right inlet meets the laminar flow channel in the plane of the top of the metal foil. The x and y directions are shown in Figure 6A. The formulation for the energy equation in the channel is as follows, wherein p is density of the fluid, c is specific heat of the fluid, $V_z$ is the speed along the channel in the z direction, T is temperature, k is the thermal conductivity of the solution flowing in the channel, and q is the heat release or absorption due to the reaction:

Integrating in y-direction over a channel depth d (y = 0 to Y= d) and defining T= 1/d ∫Tdy gives:

$$\rho c V_z d \frac{\partial \widetilde{T}}{\partial z} = k d \left( \frac{\partial^2 \widetilde{T}}{\partial x^2} + \frac{\partial^2 \widetilde{T}}{\partial z^2} \right) + k \int_{y=0}^{y=d} \frac{\partial^2 T}{\partial y^2} dy + \dot{q}(x, y, z) d$$

**Equation 6**

$$k \int_{y=0}^{y=d} \frac{\partial^2 T}{\partial y^2} dy = k \frac{\partial T}{\partial y}\bigg|_{y=d} - k \frac{\partial T}{\partial y}\bigg|_{y=0} = -q''_{y=d} + q''_{y=0}$$

**Equation 7**

Experimental results show that q~=o » q~=d' hence the loss on the PDMS side can be negligible. Next, integrating in x-direction, from x = 0 to x = w, gives the following:

$$wpcV_z d \frac{\partial \overline{\overline{T}}}{\partial z}$$

$$= kwd \frac{\partial^2 \overline{\overline{T}}}{\partial z^2} + kd \int_{x=0}^{x=w} \frac{\partial^2 T}{\partial x^2} dx + \int_{x=0}^{x=w} q''_{y=0}(x,z) dx + d \int_{x=0}^{x=w} \dot{q}(z) dx$$

**Equation 8**

The heat transfer in x-direction at the boundary is adiabatic, so:

$$k \int_{x=0}^{x=w} \frac{\partial^2 T}{\partial x^2} dx = k \frac{\partial T}{\partial x}\bigg|_{x=w} - k \frac{\partial T}{\partial x}\bigg|_{x=0} = 0$$

**Equation 9**

$$wd \rho cV_z \frac{\partial \overline{\overline{T}}}{\partial z} = kwd \frac{\partial^2 \overline{\overline{T}}}{\partial z^2} + \int_{x=0}^{x=w} q''_{y=0}(x,z) dx + d \int_{x=0}^{x=w} \dot{q}(z) dx$$

**Equation 10**

$$d \int_{x=0}^{x=w} \dot{q}(z) dx = wd \rho cV_z \frac{\partial \overline{\overline{T}}}{\partial z} - kwd \frac{\partial^2 \overline{\overline{T}}}{\partial z^2} - \int_{x=0}^{x=w} q''_{y=0}(x,z) dx$$

**Equation 11**

where q(z) is the local heat source due to the chemical reaction averaged over channel width x and depth y. Here, temperature T is also averaged over the channel width x. q~=o represents the heat flux coming from the chip surface and can be approximated by:

$$q''_{y=0} = H(x,z)\left[\overline{\overline{T}}_{chip} - \overline{\overline{T}}(z)\right]$$

**Equation 12**

wherein H(x,z) is the experimentally determined calibration heat loss coefficient for the system. Along the flow direction, between the locations Z1 and Z2, the total heat source can be obtained by integrating local heat source along z-direction. Setting f= T and rearranging gives the following equation:

$$d \int_{z_1}^{z_2} \int_{x=0}^{x=w} \dot{q}(z) dx dz$$

$$= wd\, \rho\, cV_z [T(z_2) - T(z_1)] - wd \left[ k \frac{\partial T}{\partial z}\Big|_{z_2} - k \frac{\partial T}{\partial z}\Big|_{z_1} \right]$$

$$- \int_{z_1}^{z_2} \int_{x=0}^{x=w} H(x,z)[T_{chip} - T(x,z)] dx dz$$

**Equation 13**

In this analytical approach, using the general form of energy equation, emphasis is kept on the heat source term and necessary simplifications have been done based on the previous numerical results. Equation 11 shows that enthalpy of reaction ($\Delta H = q/n_{AB}$) can be extracted based on temperature measurements of the flow field for this experimental configuration. From the enthalpy of reaction and the temperature, one can calculate further calorimetric measurements, such as the binding constant of the reaction, Gibbs free energy, change in free energy, entropy, and change in entropy from the temperature.

For example, the binding constant $K_D$ can be obtained by using the temperature profiles in energy balances using volumes bounded by $X_1$, $X_2$, $Z_1$, $Z_2$ and the channel height to determine the heat source qdue to reaction in that volume. Repeating this calculation for several volumes, $X_2$, $X_3$, $Z_1$, $Z_2$; $X_3$, $X_4$, $Z_1$, $Z_2$ ... $X_n$, $X_{n+1}$, $Z_1$, $Z_2$, will provide information on the energy released for different amounts of the reactant and products. The measured heat source due to the reaction in each volume is related to the enthalpy of reaction and the binding constant. This information is used in a combined mass balance and binding constant definition, below, to iteratively determine the binding constant using the measured heat source due to the reaction in the different volumes:

$$(n_{ML})^2 - [N_i \Delta n_L + n_{MTOT} + K_D] + (N_i \Delta n_L) n_{MTOT} = 0$$

In order to obtain accurate enthalpic measurements, the nanohole arrays should provide adequate measurement resolution while not interfering with neighboring sensors. To determine optimal nanosensor array placement, reaction simulations with nanoholes arrays in various configurations were performed. First, a model of a laminar flow channel with the dimensions mentioned with respect to Figure 5 (w =500 $\mu$m, 1=5000 $\mu$m) and depth equal to 10 $\mu$m was created in FLUENT, a computational fluid dynamics software. The continuity, Navier-Stokes, mass transport, and reaction rate equations were solved at 500,000 nodes for a steady state case of a NaOH + HCl reaction in 3D space to obtain a temperature field at the surface of the metal foil, shown in Figure 10. The model assumes a no slip condition at the walls of the channel and that the side walls arc adiabatic. The thcrmophysical properties of the fluid mixture modeled as the properties of water at 298.15 K, as the temperature change of the reaction is only $\pm5$ K and the mixture is mostly water. Figure 10 depicts the temperature map produced by this model.

Thermal boundary analysis was performed on the simulated model to analyze the temperature in the y-direction. The temperature sensed over the immediate vicinity of the metal foil was found to be 99.8610% of the average temperature normal to the channel. Since SPR sensors such as nanohole arrays only sense temperature in the near field, a thickness of $\lambda/4$ of the incident wavelength (-150 nm), this validates that near field sensing accurately represents the temperature of the entire channel.

A valid model for a temperature field of a reaction in a laminar flow channel also allows one to determine optimal nanohole array spacing. For the sensor spacing analysis, temperature, heat flux, and concentration at the metal foil surface (y =0 surface) are taken from the FLUENT model. Each term from Equation 13 is estimated at equally spaced points for multiple spacing values and the enthalpy of formation at each array may be estimated using the following equation:

$$\Delta H_{formation} = \frac{d \int_{z_1}^{z_2} \int_{x=0}^{x=w} \dot{q}(z)dxdz}{\frac{(\dot{m}_{NaCl}^{outlet} - \dot{m}_{NaCl}^{inlet})}{M_{NaCl}}}$$

**Equation 14**

So, enthalpy of formation is estimated using various array spacing algorithms and the map of the heat loss coefficient known by the simulation. The success of each spacing algorithm is determined by the accuracy of the estimated enthalpy of formation. Estimates of other known measurements, such as mass fraction and entropy, may be used alternatively or in addition to the enthalpy of formation. The procedure is repeated for different values of horizontal spacing between arrays, different inlet velocities, and different intervals in the z direction. The procedure may also be repeated for different array arrangements, diffusion rates, or other variables.

Three sensor spacing algorithms were investigated:

1. Taking the wall at x=0 as reference and placing sensors equally spaced with the sensor spacing distance;

2. Taking the center of the channel with respect to x as the reference, placing sensors equally spaced with the sensor spacing distance;

3. Taking the center of the channel with respect to x as the reference, but the first sensor will be half of the sensor spacing value away from the center (i.e. no sensor in the center).

Additionally, spacing for flow velocities of 0.0005 m/s, 0.005 m/s, and 0.05 m/s were investigated. As expected, for a channel of a given length, a larger resolution in the x-direction is required for higher flow rates since the diffusion width the x-direction is small due to the low residence time. As different reactions will have different diffusivities, the flow rate of the reagents can be varied to achieve the necessary resolution with respect to a set nanoholc array spacing.

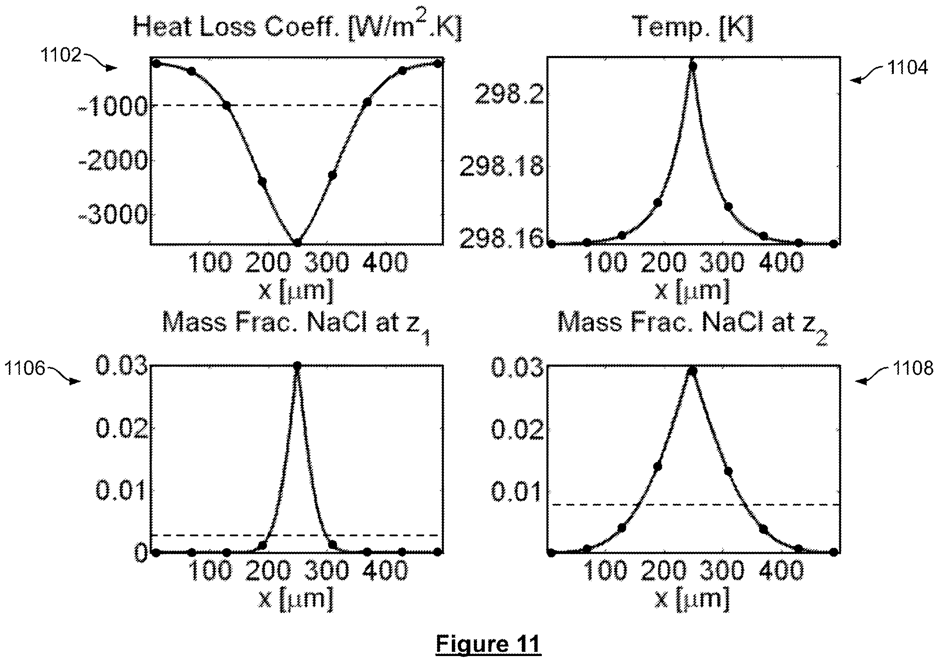

For sensor spacings ranging from 2 11m to 120 11m spaced with each of the aforementioned spacing algorithms, percent error starts deviating after 40 11m due to the shapes of the concentration, temperature, and heat loss coefficient curves as functions of x, which are similar to a Gaussian distribution. Figure 11 depicts the heat loss coefficient 1102 and temperature 1104 curves versus x at z = 50 11m for a flow rate of 0.0005 mls. The mass fraction curves 1106 and 1108 of NaCl at $Z_1$ = 50 $\mu$m and $Z_2$ = 800 $\mu$m, respectively, are below curves 1102 and 1104. A 60 $\mu$m spacing configuration can adequately represent the heat loss and temperature averages across the width of the channel, but not the mass fraction at the regions near the inlet. This behavior is due to the low values of mass diffusivity coefficient in the x direction compared to the larger values of thermal conductivity (diffusivity) constant. At certain spacing configurations, sensor locations can correspond to locations where a single point can represent the total average of the curve (the vicinity of the horizontal lines in mass fraction graphs in Figure 11). However, these locations are dependent on the curve characteristics and can change with many parameters such as the nature of reaction, and z-location. Therefore, the error analysis should be done with extra caution. The results show that this behavior starts to occur after 40 $\mu$m. Therefore, in an exemplary implementation sensors are arranged in a grid with spacing of 10 to 30 $\mu$m.

This type of modeling can be used to determine optimal sensor spacing for other flow rates. In general, higher flow rates require more closely spaced sensors in the x direction, across the width of the channel. Furthermore, the model shown can be used to examine other algorithms of spacing sensors, such as spacing wherein sensors are more concentrated toward the middle of the channel. FLUENT or another computational fluid dynamics software may be used for the modeling.

DRUG DEVELOPMENT APPLICATION

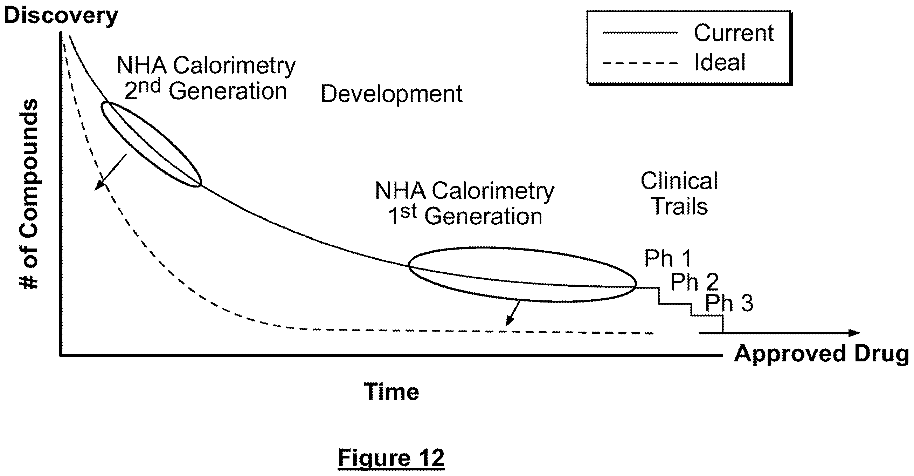

Figure 12 is a graph illustrating the trend between the number of compounds versus time in a pharmaceutical research and development process. Initially, there are a large number of compounds being investigated for a particular therapeutic target. Currently, high throughput screening is used at the start of the process, and calorimetry is generally not performed on the compounds until fairly late in the development process. One objective of the microfluidic calorimetry apparatus disclosed herein is to enable sensitive, fast, low volume calorimetry measurements so that calorimetry may be utilized earlier in the drug development, pushing the curve of number of compounds vs. time to the lower dotted line.

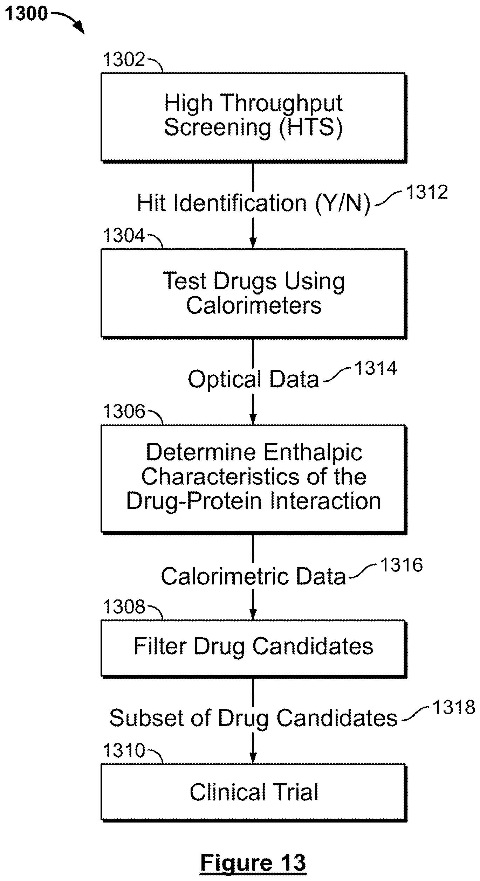

Figure 13 is a flowchart for a method of pharmaceutical research and development. First, a high-throughput screening (HTS) is performed (step 1302). HTS is an automated method for determining if a biological entity, such as a protein, cells, an embryo, etc., reacts with a compound. Current HTS systems can test up to 100,000 compounds per day, and advances in HTS screening are continually increasing their output. While HTS allows a great number of compounds to be tested, it only gives a binary result for whether a compound is active or not. Other screening methods that are practical to perform on a large number of compounds may also or alternatively be used.

After high-throughput screening and/or other screening tests, compounds are next tested using calorimetry (step 1304). Compounds that reacted in a high throughput screening are currently tested using two calorimetry techniques: differential scanning calorimetry and isothermal scanning calorimetry. The inventive microfluidic calorimetry method disclosed herein can replace both differential scanning calorimetry and isothermal scanning calorimetry with faster results and potentially using as little as one-one thousandth the reagent consumption of current calorimeters. As discussed above with respect to Figure 9, the optical data 1314 is processed to determine enthalpic characteristics of the drug-protein interaction (1306), producing calorimetric data 1316. This data is then analyzed to filter drug candidates (1308). The filtering may return all compounds with enthalpies and/or entropies in a certain range. The filtering may alternatively select a given number of compounds with best results. The calorimetric measurements may be weighted and summed to give each compound a composite score. The result of the filtering is a subset of drug candidates 1318 that are selected for a first round of a clinical trial after an institutional board review. Other preclinical studies, such as in vivo or in vitro experiments, may be required before a clinical study.

While preferable implementations of the present disclosure have been shown and described herein, it will be obvious to those skilled in the art that such implementations are provided by way of example only. It is intended that the following claims define the scope of the disclosure and that methods and structures within the scope of these claims be covered thereby.

REFERENCES

Krishnan, A. et al. "Evanescently coupled resonance in surface plasmon enhanced transmission," Optics Communications, Vol. 200, pp. 1-7, 2001.

Ji, J. et al. "High-throughput nanohole array based system to monitor multiple binding events in real time," Analytical Chemistry, Vol. 80, pp. 2491-2498, 2008.

Leach, A. et al. "Flow injection analysis in a microfluidic format," Analytical Chemistry, Vol. 75, No. 5, pp. 967-972, 2003.

Lindquist, N. et al. "Sub-micron resolution surface plasmon resonance imaging enabled by nanohole arrays with surrounding Bragg mirrors for enhanced sensitivity and isolation," Lab on a Chip, Vol. 9, pp. 382-387, 2009.

Pradere, C. et al. "Processing of temperature field in chemical microreactors with infrared thermography," QIRT, Vol. 3, No. 1, pp. 117-135, 2006.

**Claims**

1. A system for calorimetry comprising:

   a microfluidic laminar flow channel a portion of which includes a reaction chamber (715);
   a first inlet (722) coupled to the reaction chamber (715);
   a second inlet (721) coupled to the reaction chamber (715);
   a plurality of microscale sensors (502) disposed along the microfluidic reaction chamber (715);
   a first fluid injector coupled to the first inlet (722) configured to inject a first reagent bolus of a first fluid into the reaction chamber (715);
   a second fluid injector coupled to the second inlet (721) configured to inject a second fluid into the reaction chamber (715) such that the volume of a second fluid in the reaction chamber (715) is between about 4 and about 10 times greater than the volume of the first reagent bolus in the reaction chamber (715); and
   a processor configured to calculate a calorimetry value based on a plurality of temperature measurements made at each of two or more of the plurality of microscale sensors (502), the plurality of temperature measurements made over a plurality of first reagent concentrations as the first reagent bolus inter-diffuses with the second fluid and flows through the microfluidic reaction chamber (715).

2. The system of claim 1, wherein the fluid injector includes a proximal end coupled to a proximal end of the first inlet (722) and a distal end coupled to a distal end of the first inlet (722), and a valve coupled to the proximal end of the fluid injector that is configured to selectively introduce a fluid into the fluid injector to eject said first reagent bolus stored in the fluid injector into the first inlet (722).

3. The system of claim 1, wherein the first fluid injector is configured to hold between about 5 nL and about 250 nL.

4. The system of claim 1, wherein the microscale sensors (502) are nanohole arrays in a metal layer disposed below the reaction chamber (715).

5. The system of claim 4, wherein the nanohole arrays are surrounded by dielectric mirrors.

6. A method for calorimetry comprising;

   providing the calorimetry system of claim 1;
   flowing a first fluid through the first inlet (722) into the microfluidic reaction chamber (715);
   flowing the second fluid through the second inlet (721) into the microfluidic reaction chamber;
   injecting the first reagent bolus into the first inlet via the fluid injector, such that the first reagent comes into contact with the second fluid in the microfluidic reaction chamber; and
   calculating a calorimetry value based on a plurality of temperature measurements made at each of two or more of the plurality of microscale sensors (502), the plurality of temperature measurements made over a plurality of reagent concentrations as the first reagent bolus flows through the microfluidic reaction chamber.

7. The method of claim 6, further comprising selecting a portion of the calorimetry value that approximates a second calorimetry value made when a reagent of the first reagent bolus was flowing at steady state.

8. The method of claim 6, further comprising:

   injecting a second reagent bolus into the first inlet via the first fluid injector, such that the second reagent bolus comes into contact with the second fluid in the microfluidic reaction chamber; and
   calculating a second calorimetry value over a second plurality of reagent concentrations as the second reagent bolus flows through the microfluidic reaction chamber.

9. The method of claim 6, wherein calculating a calorimetry value comprises calculating at least one of an enthalpy of the reaction, a binding constant of the reaction, a Gibbs free energy value, a change in free energy, an entropy value, and a change in entropy.

10. The method of claim 6, wherein the volume of the first reagent bolus is between about 10 nL and about 1 microL.

11. The method of claim 6, further comprising selecting a flow rate of at least one of the first fluid and the second fluid based on a diffusivity of the first and second fluids.

12. The method of claim 6, wherein the first reagent bolus is between about 5 nL and about 250 nL.

13. The method of claim 6, further comprising calculating estimations of the first reagent bolus's concentration at a plurality of specific microscale sensor positions and at a plurality of specific timepoints after the fluid injector injects the first reagent bolus.

14. The method of claim 6, wherein the first reagent bolus is between about 5 nanoliters and about 50 nanoliters.

**Patentansprüche**

1. System für eine Kalorimetrie, das Folgendes umfasst:

   einen Kanal für eine mikrofluidische laminare Strömung, von dem ein Abschnitt einen Reaktionsraum (715) beinhaltet;
   einen ersten Einlass (722), der mit dem Reaktionsraum (715) gekoppelt ist;
   einen zweiten Einlass (721), der mit dem Reaktionsraum (715) gekoppelt ist;

mehrere Sensoren (502) im Mikromaßstab, die entlang dem mikrofluidischen Reaktionsraum (715) angeordnet sind;

einen ersten Fluidinjektor, der mit dem ersten Einlass (722) gekoppelt ist, der konfiguriert ist, um einen ersten Reagensbolus eines ersten Fluids in den Reaktionsraum (715) zu injizieren;

einen zweiten Fluidinjektor, der mit dem zweiten Einlass (721) gekoppelt ist, der konfiguriert ist, um ein zweites Fluid in den Reaktionsraum (715) derart zu injizieren, dass das Volumen eines zweiten Fluids in dem Reaktionsraum (715) zwischen etwa 4- und etwa 10-mal über dem Volumen des ersten Reagensbolus in dem Reaktionsraum (715) liegt; und

einen Prozessor, der konfiguriert ist, um einen Kalorimetriewert basierend auf mehreren Temperaturmessungen zu berechnen, die an jedem von zwei oder mehr der mehreren Sensoren (502) im Mikromaßstab gemacht werden, wobei die mehreren Temperaturmessungen über mehrere erste Reagenskonzentrationen gemacht werden, während der erste Reagensbolus unter das zweite Fluid diffundiert und durch den mikrofluidischen Reaktionsraum (715) strömt.

2. System nach Anspruch 1, wobei der Fluidinjektor ein proximales Ende, das mit einem proximalen Ende des ersten Einlasses (722) gekoppelt ist, und ein distales Ende, das mit einem distalen Ende des ersten Einlasses (722) gekoppelt ist, und ein Ventil beinhaltet, das mit dem proximalen Ende des Fluidinjektors gekoppelt ist, das konfiguriert ist, um ein Fluid wahlweise in den Fluidinjektor einzuführen, um den ersten Reagensbolus, der in dem Fluidinjektor gespeichert ist, in den ersten Einlass (722) auszustoßen.

3. System nach Anspruch 1, wobei der erste Fluidinjektor konfiguriert ist, um zwischen etwa 5 nL und etwa 250 nL zu halten.

4. System nach Anspruch 1, wobei die Sensoren (502) im Mikromaßstab Nanolochanordnungen in einer Metallschicht sind, die unter dem Reaktionsraum (715) angeordnet ist.

5. System nach Anspruch 4, wobei die Nanolochanordnungen von dielektrischen Spiegeln umgeben sind.

6. Verfahren für die Kalorimetrie, das Folgendes umfasst;

Bereitstellen des Kalorimetriesystems nach Anspruch 1;
Strömen eines ersten Fluids durch den ersten Einlass (722) in den mikrofluidischen Reaktionsraum (715);
Strömen des zweiten Fluids durch den zweiten Einlass (721) in den mikrofluidischen Reaktionsraum;
Injizieren des ersten Reagensbolus derart in den ersten Einlass über den Fluidinjektor, dass das erste Reagens mit dem zweiten Fluid in dem mikrofluidischen Reaktionsraum in Berührung kommt; und
Berechnen eines Kalorimetriewerts basierend auf mehreren Temperaturmessungen, die an jedem von zwei oder mehr der mehreren Sensoren (502) im Mikromaßstab gemacht werden, wobei die mehreren Temperaturmessungen über mehrere Reagenskonzentrationen gemacht werden, während der erste Reagensbolus durch den mikrofluidischen Reaktionsraum strömt.

7. Verfahren nach Anspruch 6, das ferner ein Auswählen eines Teils des Kalorimetriewerts umfasst, der einem zweiten Kalorimetriewert nahe kommt, der gemacht wurde, wenn ein Reagens des ersten Reagensbolus in einem Stationärzustand strömte.

8. Verfahren nach Anspruch 6, das ferner Folgendes umfasst:

Injizieren eines zweiten Reagensbolus derart in den ersten Einlass über den ersten Fluidinjektor, dass der zweite Reagensbolus mit dem zweiten Fluid in dem mikrofluidischen Reaktionsraum in Berührung kommt; und
Berechnen eines zweiten Kalorimetriewerts über zweite mehrere Reagenskonzentrationen, während der zweite Reagensbolus durch den mikrofluidischen Reaktionsraum strömt.

9. Verfahren nach Anspruch 6, wobei das Berechnen eines Kalorimetriewerts das Berechnen einer Enthalpie der Reaktion, einer Bindungskonstante der Reaktion, eines Werts einer freien Enthalpie, einer Änderung der freien Energie, eines Entropiewerts und/oder einer Änderung der Entropie umfasst.

10. Verfahren nach Anspruch 6, wobei das Volumen des ersten Reagensbolus zwischen etwa 10 nL und etwa 1 mikroL liegt.

**11.** Verfahren nach Anspruch 6, das ferner das Auswählen einer Strömungsgeschwindigkeit des ersten Fluids und/oder des zweiten Fluids basierend auf einem Diffusionskoeffizienten des ersten und des zweiten Fluids umfasst.

**12.** Verfahren nach Anspruch 6, wobei der erste Reagensbolus zwischen etwa 5 nL und etwa 250 nL liegt.

**13.** Verfahren nach Anspruch 6, das ferner das Berechnen von Schätzungen der Konzentration des ersten Reagensbolus an mehreren spezifischen Positionen der Sensoren im Mikromaßstab und an mehreren spezifischen Zeitpunkten umfasst, nachdem der Fluidinjektor den ersten Reagensbolus injiziert hat.

**14.** Verfahren nach Anspruch 6, wobei der erste Reagensbolus zwischen etwa 5 Nanolitern und etwa 50 Nanolitern liegt.


**Revendications**

**1.** Système destiné à la calorimétrie comprenant :

un canal microfluidique à écoulement laminaire dont une partie comporte une chambre de réaction (715) ;
une première entrée (722) accouplée à la chambre de réaction (715) ;
une seconde entrée (721) accouplée à la chambre de réaction (715) ;
une pluralité de capteurs de petite taille (502) disposés le long de la chambre de réaction microfluidique (715) ;
un premier injecteur de fluide accouplé à la première entrée (722) conçu pour injecter un premier bolus de réactif d'un premier fluide dans la chambre de réaction (715) ;
un second injecteur de fluide accouplé à la seconde entrée (721) conçu pour injecter un second fluide dans la chambre de réaction (715) de telle sorte que le volume d'un second fluide dans la chambre de réaction (715) est environ 4 à 10 fois plus important que le volume du premier bolus de réactif dans la chambre de réaction (715) ; et
un processeur conçu pour calculer une valeur calorimétrique sur la base d'une pluralité de mesures de température effectuées au niveau de chacun de deux ou plus de la pluralité de capteurs de petite taille (502), la pluralité de mesures de température étant effectuées sur une pluralité de premières concentrations de réactif pendant que le premier bolus de réactif s'interdiffuse avec le second fluide et s'écoule à travers la chambre de réaction microfluidique (715).

**2.** Système selon la revendication 1, dans lequel l'injecteur de fluide comporte une extrémité proximale accouplée à une extrémité proximale de la première entrée (722) et une extrémité distale accouplée à une extrémité distale de la première entrée (722), et une valve accouplée à l'entrée extrémité proximale de l'injecteur de fluide qui est conçue pour introduire sélectivement un fluide dans l'injecteur de fluide afin d'éjecter ledit premier bolus de réactif stocké dans l'injecteur de fluide dans la première entrée (722).

**3.** Système selon la revendication 1, dans lequel le premier injecteur de fluide est conçu pour contenir entre environ 5 nL et environ 250 nL.

**4.** Système selon la revendication 1, dans lequel les capteurs de petite taille (502) sont des réseaux de nanotrous dans une couche métallique disposée en dessous de la chambre de réaction (715).

**5.** Système selon la revendication 4, dans lequel les réseaux de nanotrous sont entourés de miroirs diélectriques.

**6.** Procédé destiné à la calorimétrie comprenant ;

la fourniture du système de calorimétrie selon la revendication 1 ;
la circulation d'un premier fluide à travers la première entrée (722) dans la chambre de réaction microfluidique (715) ;
la circulation du second fluide à travers la seconde entrée (721) dans la chambre de réaction microfluidique ;
l'injection du premier bolus de réactif dans la première entrée par l'intermédiaire de l'injecteur de fluide, de telle sorte que le premier réactif entre en contact avec le second fluide dans la chambre de réaction microfluidique ; et
le calcul d'une valeur calorimétrique sur la base d'une pluralité de mesures de température effectuées au niveau de chacun de deux ou plus de la pluralité de capteurs de petite taille (502), la pluralité de mesures de température effectuées sur une pluralité de concentrations de réactif pendant que le premier bolus de réactif s'écoule à travers la réaction microfluidique chambre.

**7.** Procédé selon la revendication 6, comprenant en outre la sélection d'une partie de la valeur calorimétrique qui se rapproche d'une seconde valeur calorimétrique effectuée lorsqu'un réactif du premier bolus de réactif s'écoulait de manière permanente.

**8.** Procédé selon la revendication 6, comprenant en outre :

l'injection d'un second bolus de réactif dans la première entrée par l'intermédiaire du premier injecteur de fluide, de telle sorte que le second bolus de réactif entre en contact avec le second fluide dans la chambre de réaction microfluidique ; et
le calcul d'une seconde valeur calorimétrique sur une seconde pluralité de concentrations de réactif pendant que le second bolus de réactif s'écoule à travers la chambre de réaction microfluidique.

**9.** Procédé selon la revendication 6, dans lequel le calcul d'une valeur calorimétrique comprend le calcul d'une enthalpie de la réaction et/ou d'une constante d'association de la réaction et/ou d'une valeur d'énergie libre de Gibbs et/ou d'un changement d'énergie libre et/ou d'une valeur d'entropie et/ou d'un changement de l'entropie.

**10.** Procédé selon la revendication 6, dans lequel le volume du premier bolus de réactif est compris entre environ 10 nL et environ 1 microL.

**11.** Procédé selon la revendication 6, comprenant en outre la sélection d'un débit du premier fluide et/ou du second fluide sur la base d'une diffusivité des premier et second fluides.

**12.** Procédé selon la revendication 6, dans lequel le premier bolus de réactif est compris entre environ 5 nL et environ 250 nL.

**13.** Procédé selon la revendication 6, comprenant en outre le calcul d'estimations de la concentration du premier bolus de réactif au niveau d'une pluralité de positions de capteurs microscopiques spécifiques et au niveau d'une pluralité de points temporels spécifiques après que l'injecteur de fluide a injecté le premier bolus de réactif.

**14.** Procédé selon la revendication 6, dans lequel le premier bolus de réactif est compris entre environ 5 nanolitres et environ 50 nanolitres.

102 ―

**Calorimetry Apparatus**

100

106 ―

**Disposable Apparatus**

104 ―

**Light Source**

110 ―
Laminar Flow Channel

112 ―
Inlets

114 ―
Metal Foil with Nanoholes

140

― Directed toward ― ― Placed in ―

108 ―

**Mounting Apparatus**

120 ―
Temperature Sensor

122 ―
Temperature Controller

124 ―
Optical Sensor

Optical Data

130 ―
― Temperature ―

132

**Figure 1**

200 ―

202

220
222
224

204

206

208

210

212

226

214

**Figure 2**

**Figure 3**

Figure 4a

Figure 4b

Figure 5

**Figure 6a**

**Figure 6b**

**Figure 6c**

Figure 7A

**FIGURE 7B**

Figure 7C    Figure 7D    Figure 7E

**800**

**802** —
Prepare
Apparatus
and Reagents

↓

**804** —
Flow Reagents
Through
Microfluidic
Channel

↓

**808** —
Sense Light
Output Through
Nanoholes

↓

**810** —
Process Data to
Determine a
Calorimetric
Measurement

<u>Figure 8</u>

**900**

**906** —
Optical Sensor Data

↓

**908**
Chemical
Concentration
Data → **902** —
Determine Local
Temperatures ← **910**
Optical
Sensor
Locations

↓

**904** —
Calculate
Calorimetric
Measurement(s)

<u>Figure 9</u>

EP 2 972 259 B1

**Figure 10**

Figure 11

EP 2 972 259 B1

**Figure 12**

1300

1302

High Throughput
Screening (HTS)

Hit Identification (Y/N) 1312

1304

Test Drugs Using
Calorimeters

Optical Data 1314

1306

Determine Enthalpic
Characteristics of the
Drug-Protein Interaction

Calorimetric Data 1316

1308

Filter Drug Candidates

Subset of Drug Candidates 1318

1310

Clinical Trial

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012097221 A

- US 7318907 B

**Non-patent literature cited in the description**

- **KRISHNAN, A. et al.** Evanescently coupled resonance in surface plasmon enhanced transmission. *Optics Communications,* 2001, vol. 200, 1-7
- **JI, J. et al.** High-throughput nanohole array based system to monitor multiple binding events in real time. *Analytical Chemistry,* 2008, vol. 80, 2491-2498
- **LEACH, A. et al.** Flow injection analysis in a microfluidic format. *Analytical Chemistry,* 2003, vol. 75 (5), 967-972

- **LINDQUIST, N. et al.** Sub-micron resolution surface plasmon resonance imaging enabled by nanohole arrays with surrounding Bragg mirrors for enhanced sensitivity and isolation. *Lab on a Chip,* 2009, vol. 9, 382-387
- **PRADERE, C. et al.** Processing of temperature field in chemical microreactors with infrared thermography. *QIRT,* 2006, vol. 3 (1), 117-135